# EUROPEAN PATENT APPLICATION

(11) **EP 4 130 007 A1**
(43) Date of publication of application: **08.02.2023**
(21) Application number: 20767705.5
(22) Date of filing: 13.04.2020
(51) Int. Cl.: C07D 493/04, H01L 51/50, H01L 51/54

(54) **HOLE TRANSPORT MATERIAL AND SYNTHESIS METHOD THEREFOR**

(30) Priority: 26.03.2020 CN 202010223433
(71) Applicant: Wuhan China Star Optoelectronics Semiconductor Display Technology Co., Ltd., Wuhan, Hubei 430079 (CN)
(72) Inventor: ZHANG, Qu, Wuhan, Hubei 430079 (CN)
(74) Representative: Stöckeler, Ferdinand
(86) International application number: PCT/CN2020/084534
(87) International publication number: WO 2021/189561

(57) **Abstract**

A hole transport material and a synthesizing method are disclosed. By introducing an aryl into a naphthofuran bromide substituted compound, a hole transport material having a HOMO/LUMO energy level and high hole mobility is synthesized, which has higher glass transition temperature, better film formation, and film stability. The current efficiency, external quantum efficiency, thermal stability, and service life of organic electroluminescent devices prepared with this material as a hole transport layer have been greatly improved.

## Description

### BACKGROUND OF INVENTION

### Field of Invention

The present application relates to a technical field of materials for organic electroluminescent diodes, in particular to a hole transport material and a synthesizing method thereof.

### Description of Prior Art

It is known that organic light-emitting diodes (OLEDs) have attracted attention from many researchers, due to their huge application prospects and advantages, such as self-illumination without the need for a backlight, high light-emitting efficiency, and so on. In order to improve luminous efficiency and extend a service life, development and research of light-emitting devices are increasingly active.

Brightness and performance of the organic electroluminescent devices are related to matching of energy levels of a hole transport layer and its adjacent functional layer, and balance of electrons and holes injected by carriers, etc. A hole transport material must have high hole mobility rate, appropriate HOMO/LUMO energy level, and thermal stability at the same time. A difference in energy level between the hole transport layer and its adjacent functional layer is also often considered to have a significant correlation with the efficiency and stability of the device. If the difference in HOMO energy level between the hole transport layer and the hole injection layer is too large, initial voltage of the device will increase, resulting in a reduced service life of the device. The large difference in HOMO energy level between the hole transport layer and the main material of the light-emitting layer also prevents holes from being transported to the light-emitting layer. The imbalance in injection between electrons and holes and the difference in mobility therebetween prevent the carriers injected from opposite poles from being effectively confined in the light-emitting layer to form excitons, causing some of the excess carriers to reach the electrode, resulting in quenching of light emission at the electrode, and reducing the luminous efficiency of the device.

When preparing an organic light-emitting diode (OLED) element, after vacuum vapor depositing organic materials on the substrate, an organic film should exhibit an amorphous film structure. After a long period of operation, the organic film in the device may change from an amorphous state to a partially crystalline state, which causes some physical properties to change, resulting in degradation of the device. A difficulty of the changing from an amorphous state to a partially crystalline state is mainly related to a glass transition temperature (Tg) of the film material, and the higher the glass transition temperature, the more stable the properties of the film and the less likely it is to crystallize. The hole transport material with a lower glass transition temperature is not easy to form a stable amorphous structure during the vapor deposition process of the device, and the film thus formed is prone to pinholes.

### SUMMARY OF INVENTION

In order to solve the problems in the prior art, an object of the present application is to provide a hole transport material with a higher hole transport rate, a higher glass transition temperature, and a suitable HOMO/LUMO energy level and a synthesizing method thereof.

The present application provides a hole transport material, having a structural formula as shown in Formula (1): 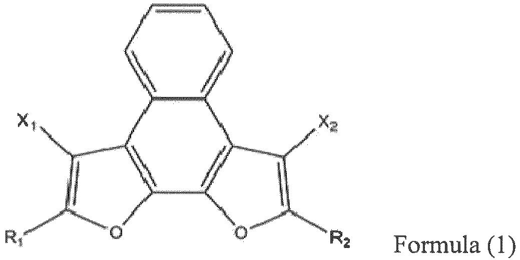 wherein R1 and R2 are the same or different and each independently selected from aryl or deuterated aryl having 30 or less carbon atoms; and X1 and X2 are the same or different and each selected from hydrogen or deuterium.

In some embodiments, each of R1 and R2 is independently selected from one of six-membered ring hydrocarbon aryl, six-membered ring heteroaryl, five-membered heterocyclic substituent, and deuterated analogues of six-membered ring hydrocarbon aryl, six-membered ring heteroaryl, and five-membered heterocyclic substituent.

In some embodiments, each of R1 and R2 is independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

In some embodiments, each of R1 and R2 is independently selected from one of the following structural formulas:

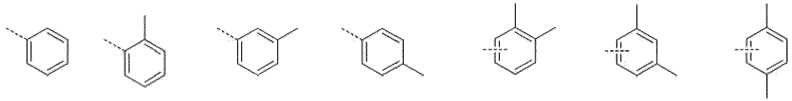

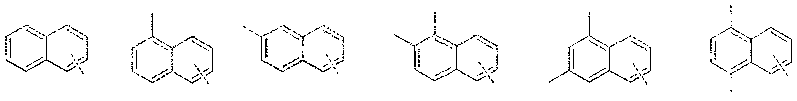

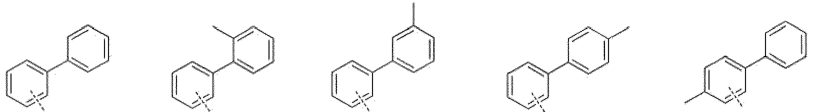

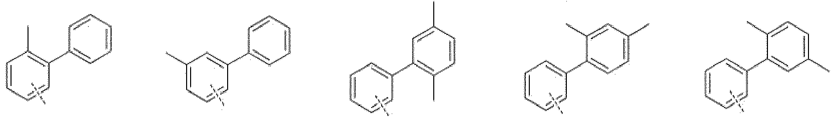

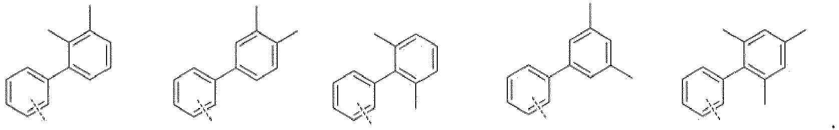

The present application also provides method of synthesizing the above-described hole transport material, including the steps of: dissolving a first reactant, a second reactant, and a catalyst in a solvent under protection of an inert gas, followed by heating for reaction, quenching, separating and purifying, to obtain the hole transport material,
wherein, when R1 and R2 are the same, the first reactant has a structural formula shown in Formula (2): 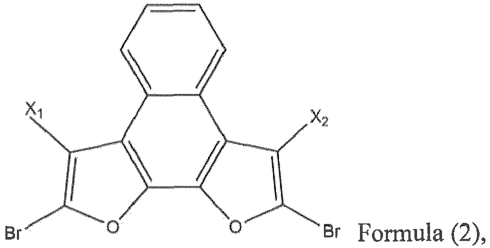 the second reactant has a structural formula shown in Formula (3): 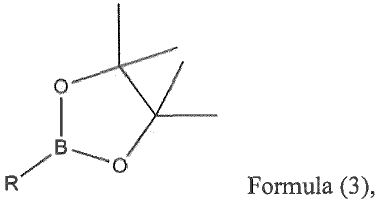 and
R is selected from R1 or R2; and
wherein, when R1 and R2 are different, the first reactant has a structural formula shown in Formula (4): 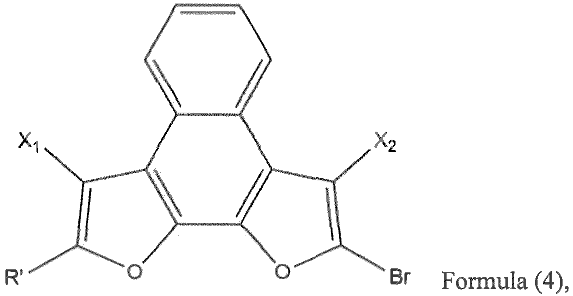
the second reactant has a structural formula shown in Formula (3), and
R' and R are different, each selected from R1 or R2.

In some embodiments, each of R1 and R2 is independently selected from one of six-membered ring hydrocarbon aryl, six-membered ring heteroaryl, five-membered heterocyclic substituent, and deuterated analogues of six-membered ring hydrocarbon aryl, six-membered ring heteroaryl, and five-membered heterocyclic substituent.

In some embodiments, each of R1 and R2 is independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

In some embodiments, each of R1 and R2 is independently selected from one of the following structural formulas:

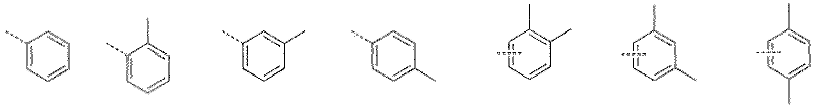

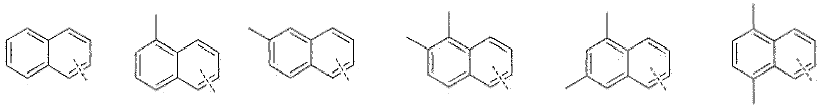

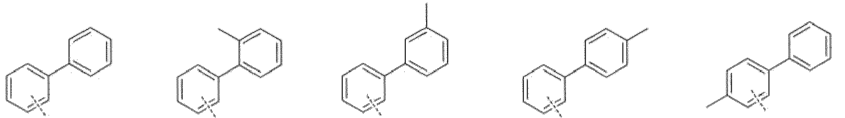

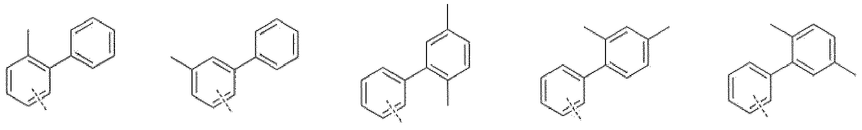

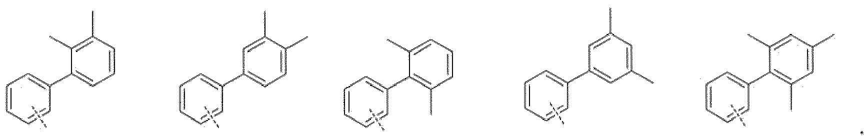

In some embodiments, the catalyst is tetratriphenylphosphor palladium and potassium carbonate, and the first reactant, the second reactant, tetratriphenylphosphor palladium, and potassium carbonate have a molar ratio of 5: (10-12): (0.5-0.6): (10-12).

In some embodiments, the solvent is a mixture of anhydrous anaerobic toluene, deoxygenated ethanol, and deoxygenated distilled water, wherein the anhydrous anaerobic toluene, deoxygenated ethanol, and deoxygenated distilled water have a volume ratio of 5: (2.5-3): (1-2).

In some embodiments, a ratio of an amount of the first reactant to a volume of the anhydrous anaerobic toluene is 0.1 to 0.2 mol/L.

In some embodiments, the heating is performed at a temperature of 100°C to 110°C, and the reaction is performed for a time period of 40 to 50 hours.

The present application also provides an organic electroluminescent device, including an anode layer, a hole injection layer, a hole transport layer, a light-emitting layer, and a cathode layer arranged sequentially from bottom to top, wherein the hole transport layer including the hole transport material according to claim 1.

In some embodiments, each of R1 and R2 is independently selected from one of six-membered ring hydrocarbon aryl, six-membered ring heteroaryl, five-membered heterocyclic substituent, and deuterated analogues of six-membered ring hydrocarbon aryl, six-membered ring heteroaryl, and five-membered heterocyclic substituent.

In some embodiments, each of R1 and R2 is independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

In some embodiments, each of R1 and R2 is independently selected from one of the following structural formulas: 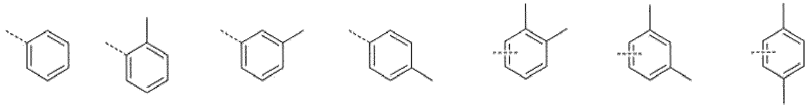 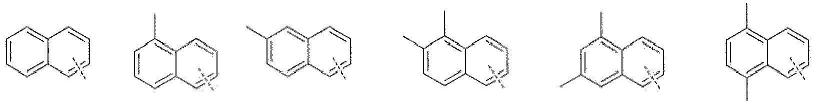 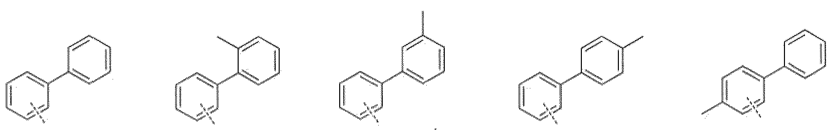 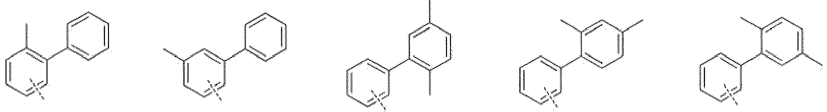 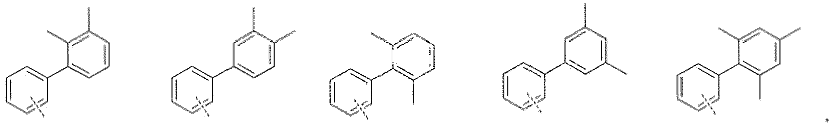
(1) In this application, by introducing an aryl into a naphthofuran bromide substituted compound, the synthesized compound has a larger planar structure and is a hole transport material having a HOMO/LUMO energy level matched to that of its adjacent functional layer, high hole mobility, and high glass transition temperature.
(2) A compound with a deuterated naphthofuran ring is used as a first reactant, and the synthesized hole transport material has higher stability.
(3) The synthesizing method of the hole transport material is simple, the reaction conditions are easy to control and implement, and the yield of the target product is high, such that it has good industrialization prospects.
(4) The synthesized naphthofuran derivatives with appropriate HOMO/LUMO energy levels can be applied to a hole transport layer in an organic electroluminescent device to better adjust a carrier balance, such that current efficiency of the device, external quantum efficiency, and its service life can be greatly improved.

### BRIEF DESCRIPTION OF DRAWINGS

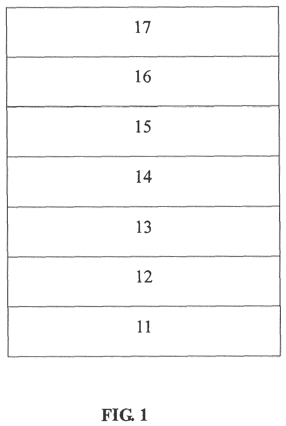

FIG. 1 is a schematic structural diagram of an organic electroluminescent device provided by an embodiment of the present application.

### DETAILED DESCRIPTION OF PREFERRED EMBODIMENTS

First, the definition and clarification of terms are proposed, followed by description of the hole transport material with a structural formula as shown in Formula (1), and finally, embodiments.

### 1. Definition and clarification of terms

Before presenting the details of the following embodiments, some terms are defined or clarified.

"Aryl" may be a single ring or multiple rings (two or more rings) fused or covalently linked together; "hydrocarbon aryl" means one or more aromatic rings having only carbon atoms thereon; "heteroaryl" means at least one aromatic ring having one or more heteroatoms thereon, and "five-membered heterocyclic substituent" means a five-membered cyclic compound having one or more heteroatoms thereon.

"Number of carbon atoms" means a total number of carbon atoms on R1 and R2. "deuterated aryl" means aryl having at least one hydrogen that has been replaced by deuterium, and "deuterated analogue" refers to a structural analogue of one or more compounds or groups in which hydrogen has been replaced by deuterium.

All groups can be unsubstituted or substituted. The structural formula as shown below 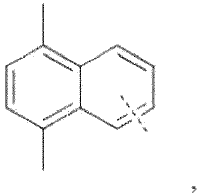 wherein the solid line represents a position at which the group is substituted by substituent, and the dashed line means that the group can be bonded at any available position on one or more rings.

In addition, in the structure represented by the following structural formula, unless otherwise defined, all technical and scientific terms used herein have the same meaning as commonly understood by those of ordinary skill in the art to which this application belongs. Although methods and materials similar or equivalent to those described herein can be used in the practice or testing of embodiments of the present application, suitable methods and materials are described below. In addition, the materials, methods, and embodiments are merely exemplary and are not intended to be limiting.

2. A hole transport material having a structural formula as shown in Formula (1): 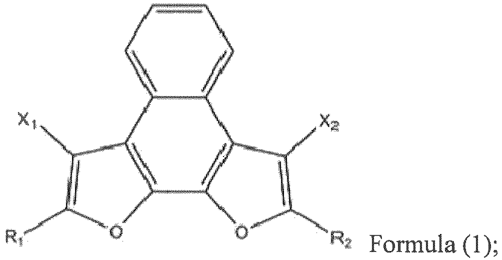 wherein R1 and R2 are the same or different and each independently selected from aryl or deuterated aryl having 30 or less carbon atoms; and X1 and X2 are the same or different and each selected from hydrogen or deuterium.

In some embodiments, each of R1 and R2 is independently selected from six-membered ring hydrocarbon aryl.

In some embodiments, each of R1 and R2 is independently selected from six-membered ring heteroaryl or five-membered ring heteroaryl.

In some embodiments, each of R1 and R2 is independently selected from a deuterated analogue of six-membered ring hydrocarbon aryl, six-membered ring heteroaryl, or five-membered ring heteroaryl. The five-membered ring heteroaryl may have 1 to 2 ring-forming heteroatoms which are selected from at least one nitrogen atoms, sulfur atoms, and oxygen atoms.

In some embodiments, each of R1 and R2 is independently selected from phenyl, biphenyl, or naphthyl.

In some embodiments, each of R1 and R2 are independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

In some embodiments, each of R1 and R2 is independently selected from one of the following structural formulas: 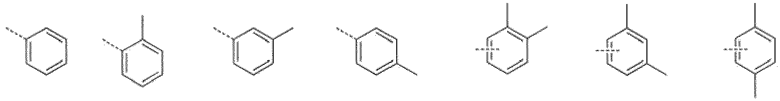 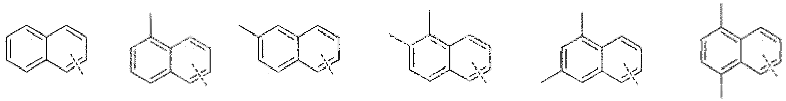 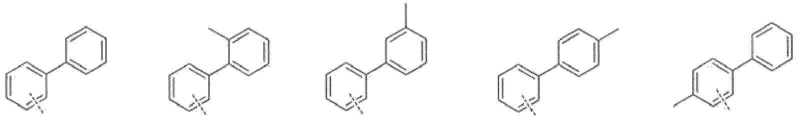 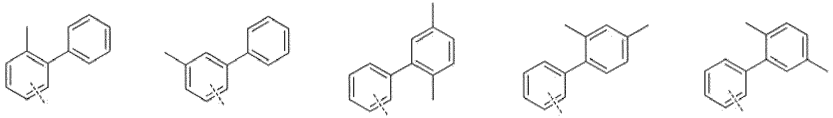 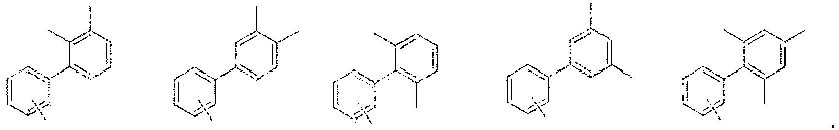 In some embodiments, a method of synthesizing the hole transport material includes the steps of: dissolving a first reactant, a second reactant, and a catalyst in a solvent under protection of an inert gas, followed by heating for reaction, quenching, separating and purifying, to obtain the hole transport material,
wherein, when R1 and R2 are the same, the first reactant has a structural formula shown in Formula (2): 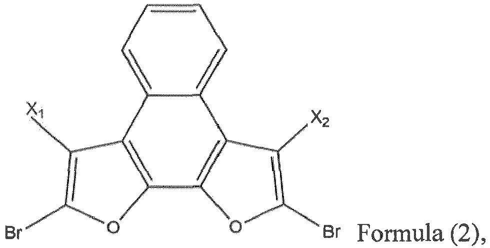 the second reactant has a structural formula shown in Formula (3): 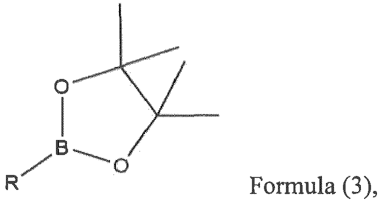 and
R is selected from R1 or R2; and
wherein, when R1 and R2 are different, the first reactant has a structural formula shown in Formula (4): 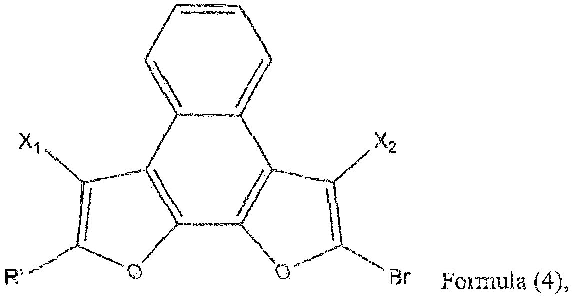
the second reactant has a structural formula shown in Formula (3), and
R' and R are different, each selected from R1 or R2.

In some embodiments, the catalyst is tetratriphenylphosphor palladium and potassium carbonate, and the first reactant, the second reactant, tetratriphenylphosphor palladium, and potassium carbonate have a molar ratio of 5: (10-12): (0.5-0.6): (10-12).

In some embodiments, the solvent is a mixture of anhydrous anaerobic toluene, deoxygenated ethanol, and deoxygenated distilled water, wherein the anhydrous anaerobic toluene, deoxygenated ethanol, and deoxygenated distilled water have a volume ratio of 5: (2.5-3): (1-2), a ratio of an amount of the first reactant to a volume of the anhydrous anaerobic toluene is 0.1 to 0.2 mol/L, and the heating is performed at a temperature of 100°C to 110°C, and the reaction is performed for a time period of 40 to 50 hours.

3. The following further describes the specific implementation of the present application in conjunction with specific embodiments 1 to 6 and the drawings, but the implementation of the present application is not limited thereto.

### Example 1

This example provides a hole transport material made of Compound 1 having a structural formula represented by Formula (1-1):

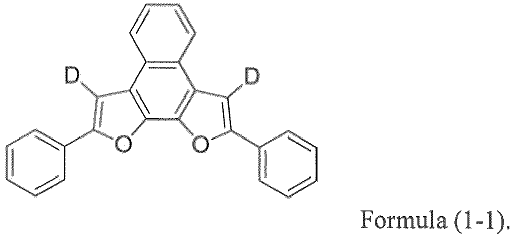

In this example, Compound 1 was synthesized according to the following reaction scheme:

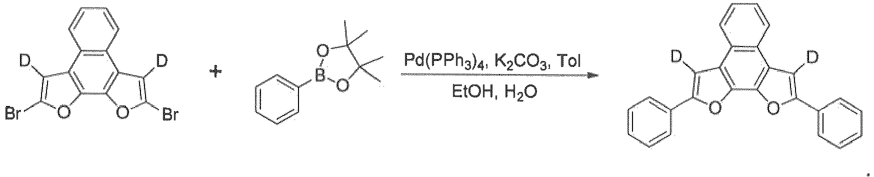

### The method of synthesizing Compound 1 includes:

A first reactant (the structure as shown in Formula (2-1), 1.84 g, 5 mmol), a second reactant (the structure as shown in Formula (3-1), 2.45 g, 12 mmol), tetratriphenylphosphine palladium (578 mg, 0.5 mmol), and potassium carbonate (1.66 g, 12 mmol) were added into a 250 mL two-necked flask, then argon gas was injected therein three times to remove the air in the two-neck flask, then 50 mL of anhydrous anaerobic toluene, 25 mL of deoxygenated ethanol, and thereafter 10 mL of deoxygenated distilled water was added therein under protection of argon gas for a reaction at 110 °C for 48 hours, followed by cooling to room temperature, and a quenching reaction with 200 mL of saturated saline. The so-called quenching reaction means terminating the reaction. The organic extracts were extracted three times with dichloromethane, combined, dried, and filtered, and then purified by silica gel column chromatography (n-hexane: ethyl acetate = 3: 1), to obtain white powder Compound 1 (1.34 g, yield 74%).

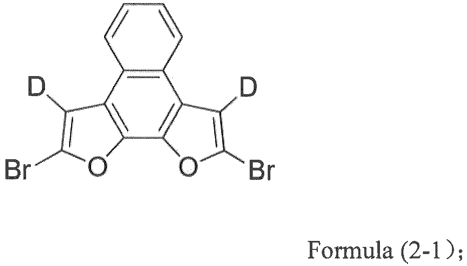

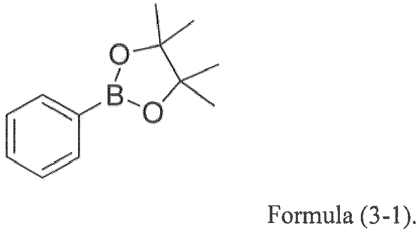

### Example 2

This example provides a hole transport material made of Compound 2 having a structural formula represented by Formula (1-2):

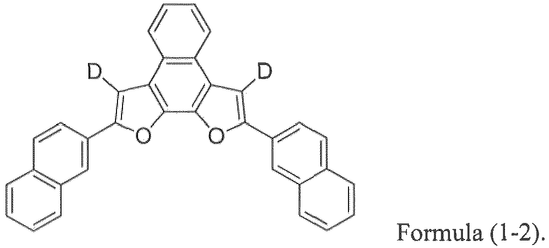

In this example, Compound 2 was synthesized according to the following reaction scheme:

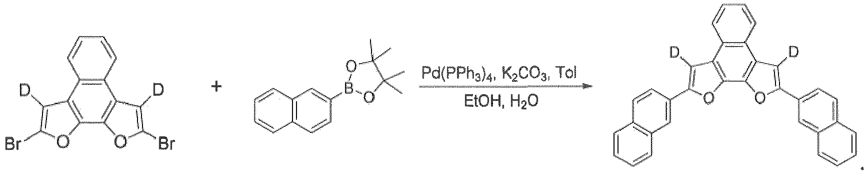

### The method of synthesizing Compound 2 includes:

A first reactant (the structure as shown in Formula (2-2), 1.84 g, 5 mmol), a second reactant (the structure as shown in Formula (3-2), 3.05 g, 12 mmol), tetratriphenylphosphine palladium (578 mg, 0.5 mmol), and potassium carbonate (1.66 g, 12 mmol) were added into a 250 mL two-necked flask, then argon gas was injected therein three times to remove the air in the two-neck flask, then 50 mL of anhydrous anaerobic toluene, 25 mL of deoxygenated ethanol, and thereafter 10 mL of deoxygenated distilled water was added therein under protection of argon gas for a reaction at 110 °C for 48 hours, followed by cooling to room temperature, and a quenching reaction with 200 mL of saturated saline. The so-called quenching reaction means terminating the reaction. The organic extracts were extracted three times with dichloromethane, combined, dried, and filtered, and then purified by silica gel column chromatography (n-hexane: ethyl acetate = 3: 1), to obtain white powder Compound 2 (1.62 g, yield 70%).

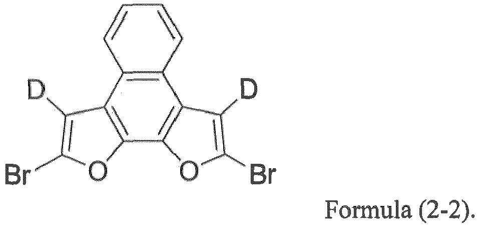

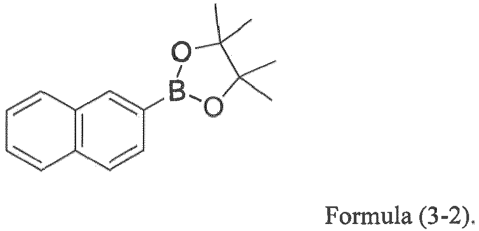

### Example 3

This example provides a hole transport material made of Compound 3 having a structural formula represented by Formula (1-3):

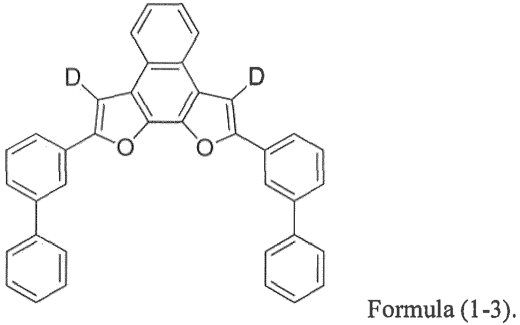

In this example, Compound 3 was synthesized according to the following reaction scheme:

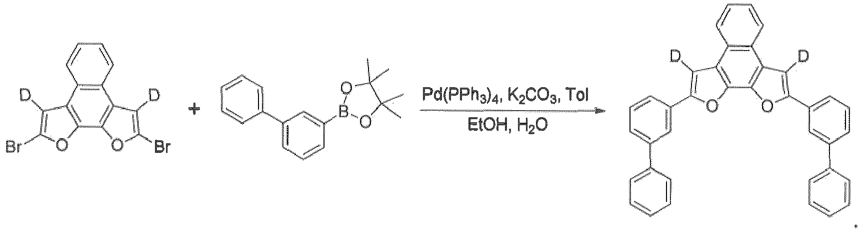

### The method of synthesizing Compound 3 includes:

A first reactant (the structure as shown in Formula (2-3), 1.84 g, 5 mmol), a second reactant (the structure as shown in Formula (3-3), 3.36 g, 12 mmol), tetratriphenylphosphine palladium (578 mg, 0.5 mmol), and potassium carbonate (1.66 g, 12 mmol) were added into a 250 mL two-necked flask, then argon gas was injected therein three times to remove the air in the two-neck flask, then 50 mL of anhydrous anaerobic toluene, 25 mL of deoxygenated ethanol, and thereafter 10 mL of deoxygenated distilled water was added therein under protection of argon gas for a reaction at 110 °C for 48 hours, followed by cooling to room temperature, and a quenching reaction with 200 mL of saturated saline. The so-called quenching reaction means terminating the reaction. The organic extracts were extracted three times with dichloromethane, combined, dried, and filtered, and then purified by silica gel column chromatography (n-hexane: ethyl acetate = 3: 1), to obtain white powder Compound 3 (1.82 g, yield 71%).

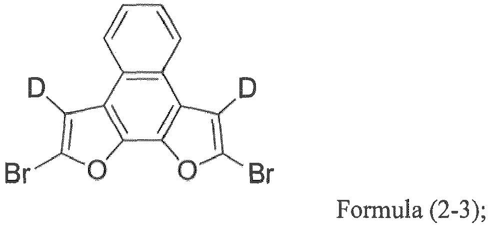

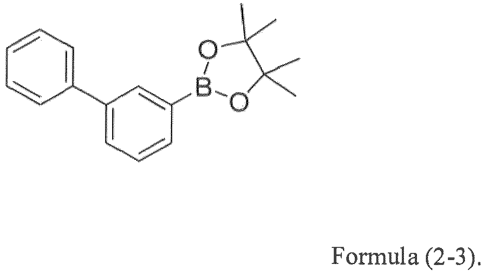

### Example 4

This example provides a hole transport material made of Compound 4 having a structural formula represented by Formula (1-4):

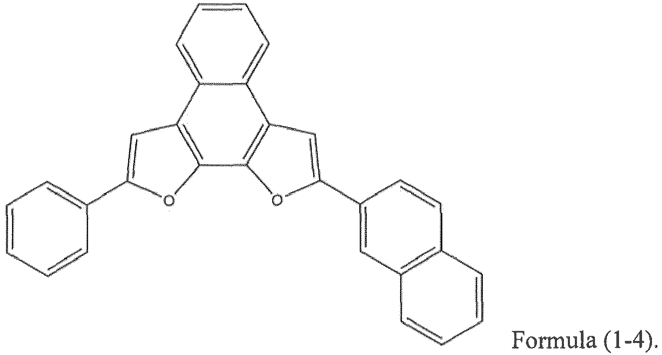

In this example, Compound 4 was synthesized according to the following reaction scheme:

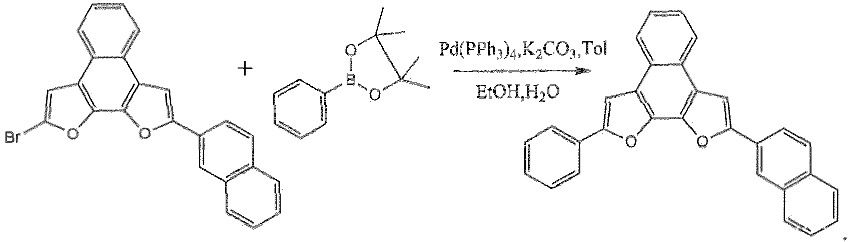

### The method of synthesizing Compound 4 includes:

A first reactant (the structure as shown in Formula (2-4), 2.21 g, 5 mmol), a second reactant (the structure as shown in Formula (3-4), 2.45 g, 12 mmol), tetratriphenylphosphine palladium (578 mg, 0.5 mmol), and potassium carbonate (1.66 g, 12 mmol) were added into a 250 mL two-necked flask, then argon gas was injected therein three times to remove the air in the two-neck flask, then 50 mL of anhydrous anaerobic toluene, 25 mL of deoxygenated ethanol, and thereafter 10 mL of deoxygenated distilled water was added therein under protection of argon gas for a reaction at 110 °C for 48 hours, followed by cooling to room temperature, and a quenching reaction with 200 mL of saturated saline. The so-called quenching reaction means terminating the reaction. The organic extracts were extracted three times with dichloromethane, combined, dried, and filtered, and then purified by silica gel column chromatography (n-hexane: ethyl acetate = 3: 1), to obtain white powder Compound 4 (1.48 g, yield 72%).

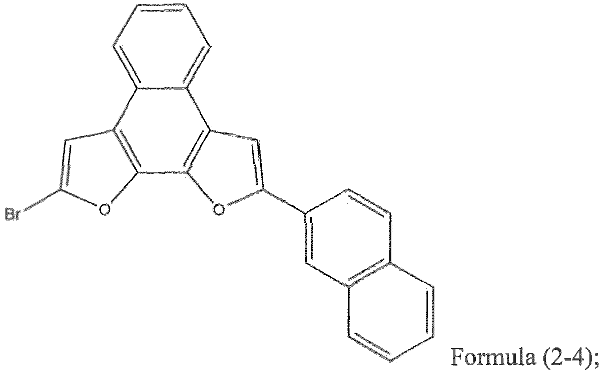

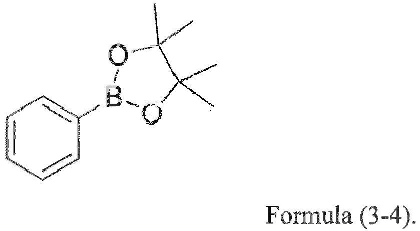

### Example 5

Mass spectrometry, hole mobility, HOMO energy level, LOMO energy level, triplet energy level, and glass transition temperature of Compound 1, Compound 2, Compound 3 and Compound 4 prepared in Examples 1 to 4 were tested. The test results are shown in Table 1 below.

**Table 1 shows the results of mass spectrometry, hole mobility, electrochemical energy level, triplet energy level, and glass transition temperature of Compound 1 to Compound 4.**

| | MS (EI) m/z : [M]+ | hole mobility (cm² /V·s ) | HOMO (eV) | LUMO (eV) | T1 (eV) | glass transition temperature ( °C ) |
|---|---|---|---|---|---|---|
| Compound 1 | 362.13 | 3.4E-4 | -5.69 | -2.24 | 2.70 | 155 |
| Compound 2 | 462.16 | 5.5E-4 | -5.67 | -2.34 | 2.67 | 168 |
| Compound 3 | 514.19 | 3.8E-4 | -5.69 | -2.46 | 2.69 | 161 |
| Compound 4 | 360.40 | 3.4E-4 | -5.70 | -2.25 | 2.70 | 154 |

It can be seen from Table 1 that the molecular weights of Compound 1 to Compound 4 after mass spectrometry analysis are consistent with their respective standard masses, that is, Compound 1 to Compound 4 are the designed target products.

The Compound 1 to Compound 4 prepared in the examples of the present application, as hole transport materials, have suitable HOMO and LUMO energy levels, and compared to an appropriate range of energy level (-5.20 eV to -5.80 eV) of a traditional hole transport layer that is matched with the energy levels of a hole injection layer and a light-emitting layer, the HOMO energy levels of Compound 1 to Compound 4 are all within this appropriate range. The HOMO energy level of the hole transport material is the same as or higher than the HOMO energy level of the main material of the light-emitting layer, and holes can be properly transported to the light-emitting layer to improve the service life and efficiency.

The hole mobility of traditional hole materials is 1.0-2.0 E-4 c m²/V • s. Compared with the traditional materials, the hole transport materials prepared in the examples of the present application have a higher hole mobility.

Meanwhile, in the structures of Compounds 1 to 4, due to the introduction of phenyl, biphenyl or naphthyl onto a naphthofuran skeleton, the conjugated system expands, and these compounds have a higher triplet energy level, which can confine the triplet excitons of the host in the light-emitting layer, thereby effectively forming triplet-triplet fusion (TTF) or phosphorescence, blocking the diffusion of triplet excitons in the light-emitting layer, reducing energy loss, and improving luminous efficiency.

The glass transition temperature is a key indicator that affects the film formation and film stability of the material. The glass transition temperature of traditional hole transport materials is generally 60 to 100 °C. As can be seen from Table 2, Compound 1 to Compound 4 have higher glass transition temperature, higher thermal stability, and they are easier to form a stable amorphous structure during vapor depositing the device, which can meet the requirements of film formation and film stability of the OLED device preparation process.

### Example 6

In this example, Compound 1 to Compound 4 and a traditional hole transport material were applied to the preparation of organic electroluminescent devices, respectively, to obtain the organic electroluminescent device 1, organic electroluminescent device 2, organic electroluminescent device 3, organic electroluminescent device 4, and organic electroluminescent device 5. The schematic structural diagram of each of these organic electroluminescent devices is shown in FIG. 1, which includes an anode layer 11, a hole injection layer 12, a hole transport layer 13, a light-emitting layer 14, an electron transport layer 15, an electron injection layer 16, and a cathode layer 17 arranged sequentially from bottom to top. The anode layer 11 was made of ITO, the light-emitting layer 14 was a polymer light-emitting layer, the electron injection layer 16 was made of Yb, and the cathode layer 17 was made of Ag/Mg.

The organic electroluminescent devices 1 to 5 were subjected to current-voltage-chromaticity test and service life test respectively. The test results are shown in Table 2 below.

CIEX and CIEY refer to the x and y color coordinates according to the C.I.E chromaticity scale (International Lighting Commission, 1931); LT95 lifetime refers to a decay time of the LT95 lifetime at 3000 illuminance.

**Table 2 shows the results of the current-voltage-chromaticity test and service life test of the organic electroluminescent devices 1 to 5.**

| Device | Hole transport layer | Maximum current efficiency (cd/A) | (CIEx, CIEy) | Maximum external quantum efficiency (%) | LT95 lifetime (hr) |
|---|---|---|---|---|---|
| 1 | Compound 1 | 58.5 | (0.685,0.315) | 42.8% | 2000 |
| 2 | Compound 2 | 61.3 | (0.684,0.316) | 44.9% | 2000 |
| 3 | Compound 3 | 58.0 | (0.686,0.314) | 43.9% | 2000 |
| 4 | Compound 4 | 58.3 | (0.685,0.314) | 42.7% | 1900 |
| 5 | traditional hole transport material | 55.0 | (0.684,0.317) | 41.3% | 1500 |

It can be seen from Table 2 that the performance test of the devices uses the organic electroluminescent device 5 as a reference, and the current efficiency of the organic electroluminescent device 5 prepared by a traditional hole transport material was 55.0 cd/A; the CIE color coordinate was (0.684, 0.317); The maximum external quantum efficiency was 41.3%; the decay time of the LT95 lifetime at 3000 illuminance was 1500 hr. Compared with the organic electroluminescent device 5 prepared by the traditional hole transport material, the organic electroluminescent devices 1 to 4 prepared by Compound 1 to Compound 4 as the hole transport layers have achieved great improvement in the highest current efficiency, the maximum external quantum efficiency, and the service life. Therefore, the compounds prepared in the examples of the present application can be applied to the preparation of organic electroluminescent devices and have good application effects as well as industrialization prospects.

In summary, although the present application has been disclosed as preferred embodiments above, the above preferred embodiments are not intended to limit the present application. Those of ordinary skill in the art can make various changes without departing from the spirit and scope of the present application Such changes and retouching, so the scope of protection of this application shall be subject to the scope defined by the claims.

## Claims

1. A hole transport material, having a structural formula as shown in Formula (1): 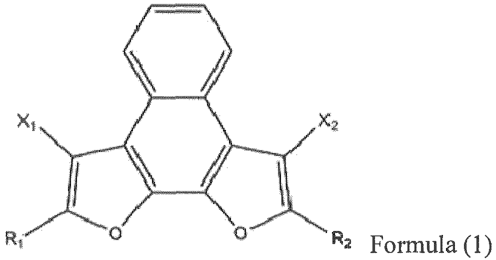 wherein R₁ and R₂ are the same or different and each independently selected from aryl or deuterated aryl having 30 or less carbon atoms; and X₁ and X₂ are the same or different and each selected from hydrogen or deuterium.

2. The hole transport material according to claim 1, wherein each of R₁ and R₂ is independently selected from one of six-membered ring hydrocarbon aryl, six-membered ring heteroaryl, five-membered heterocyclic substituent, and deuterated analogues of six-membered ring hydrocarbon aryl, six-membered ring heteroaryl, and five-membered heterocyclic substituent.

3. The hole transport material according to claim 1, wherein each of R₁ and R₂ is independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

4. The hole transport material according to claim 1, wherein each of R₁ and R₂ is independently selected from one of the following structural formulas:

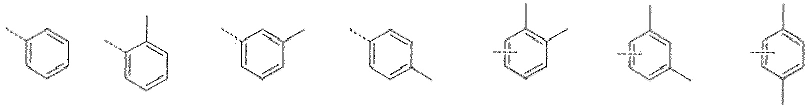

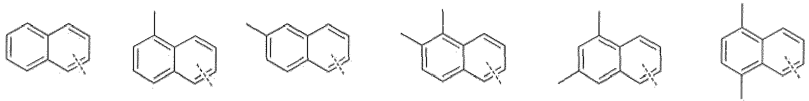

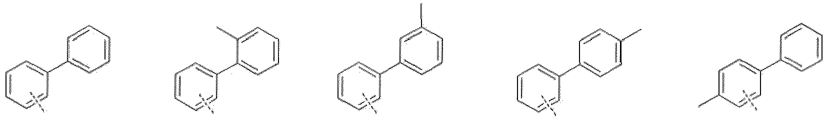

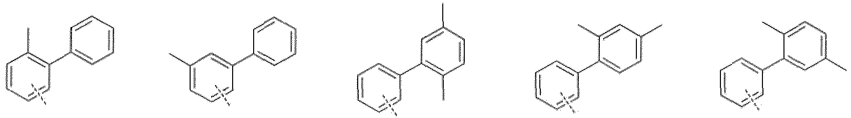

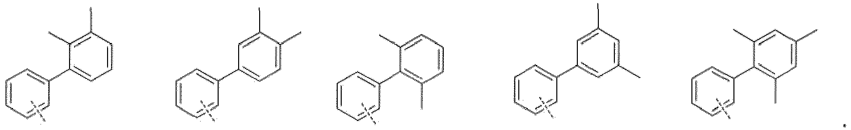

5. A method of synthesizing the hole transport material according to claim 1, comprising the steps of: dissolving a first reactant, a second reactant, and a catalyst in a solvent under protection of an inert gas, followed by heating for reaction, quenching, separating and purifying, to obtain the hole transport material,
wherein, when R₁ and R₂ are the same, the first reactant has a structural formula shown in Formula (2): 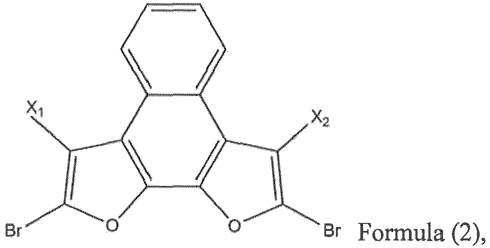 the second reactant has a structural formula shown in Formula (3): 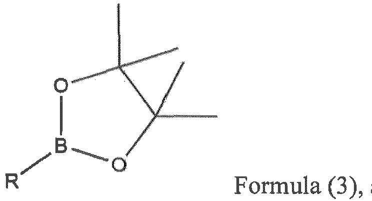 and
R is selected from R1 or R2; and
wherein, when R₁ and R₂ are different, the first reactant has a structural formula shown in Formula (4): 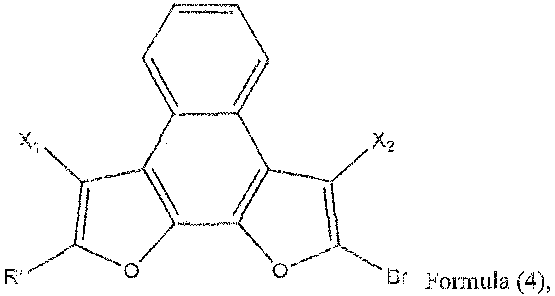
the second reactant has a structural formula shown in Formula (3), and
R' and R are different, each selected from R₁ or R₂.

6. The synthesizing method according to claim 5, wherein each of R1 and R2 is independently selected from one of six-membered ring hydrocarbon aryl, six-membered ring heteroaryl, five-membered heterocyclic substituent, and deuterated analogues of six-membered ring hydrocarbon aryl, six-membered ring heteroaryl, and five-membered heterocyclic substituent.

7. The synthesizing method according to claim 5, wherein each of R1 and R2 is independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

8. The synthesizing method according to claim 5, wherein each of R1 and R2 is independently selected from one of the following structural formulas:

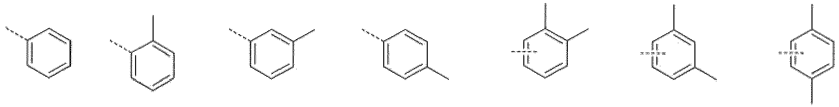

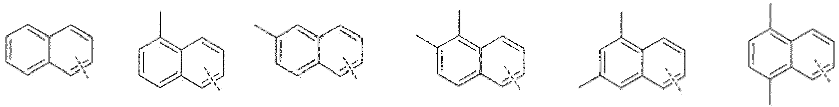

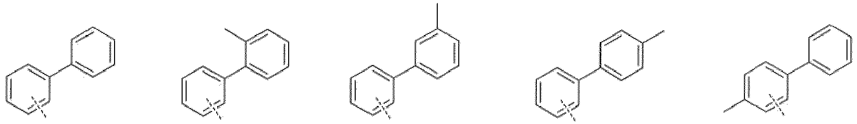

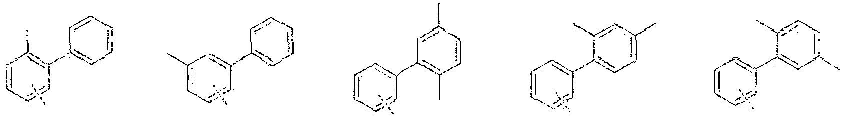

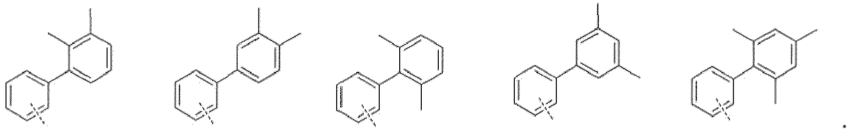

9. The synthesizing method according to claim 5, wherein the catalyst is tetratriphenylphosphor palladium and potassium carbonate, and the first reactant, the second reactant, tetratriphenylphosphor palladium, and potassium carbonate have a molar ratio of 5: (10-12): (0.5-0.6): (10-12).

10. The synthesizing method according to claim 9, wherein the solvent is a mixture of anhydrous anaerobic toluene, deoxygenated ethanol, and deoxygenated distilled water, wherein the anhydrous anaerobic toluene, deoxygenated ethanol, and deoxygenated distilled water have a volume ratio of 5: (2.5-3): (1-2).

11. The synthesizing method according to claim 10, wherein a ratio of an amount of the first reactant to a volume of the anhydrous anaerobic toluene is 0.1 to 0.2 mol/L.

12. The synthesizing method according to claim 5, wherein the heating is performed at a temperature of 100°C to 110°C, and the reaction is performed for a time period of 40 to 50 hours.

13. An organic electroluminescent device, comprising an anode layer, a hole injection layer, a hole transport layer, a light-emitting layer, and a cathode layer arranged sequentially from bottom to top, wherein the hole transport layer comprising the hole transport material according to claim 1.

14. The organic electroluminescent device according to claim 13, wherein each of R1 and R2 is independently selected from one of six-membered ring hydrocarbon aryl, six-membered ring heteroaryl, five-membered heterocyclic substituent, and deuterated analogues of six-membered ring hydrocarbon aryl, six-membered ring heteroaryl, and five-membered heterocyclic substituent.

15. The organic electroluminescent device according to claim 13, wherein each of R1 and R2 is independently selected from substituted or unsubstituted phenyl, substituted or unsubstituted biphenyl, substituted or unsubstituted naphthyl, and deuterated analogues of substituted or unsubstituted phenyl, substituted or unsubstituted biphenyl, and substituted or unsubstituted naphthyl.

16. The organic electroluminescent device according to claim 13, wherein each of R1 and R2 is independently selected from one of the following structural formulas:
